# EUROPEAN PATENT SPECIFICATION

(11) **EP 3 261 452 B1**
(45) Date of publication and mention of the grant of the patent: **02.01.2019**
(21) Application number: 16705781.9
(22) Date of filing: 22.02.2016
(51) Int. Cl.: A23C 19/032, C12R 1/225, C12N 1/20

(54) **NOVEL THERMOTOLERANT LACTOBACILLUS**
NEUARTIGER WÄRMETOLERANTER LACTOBACILLUS
NOUVEAU LACTOBACILLUS THERMOTOLÉRANT

(30) Priority: 23.02.2015 EP 15156057
(43) Date of publication of application: 03.01.2018
(73) Proprietor: Chr. Hansen A/S, 2970 Hoersholm (DK)
(72) Inventor: BUCHHORN, Gaelle Lettier, 2830 Virum (DK); VANELL, Dina, 2100 Copenhagen Ø (DK); SOERENSEN, Kim Ib, 3520 Farum (DK); OEREGAARD, Gunnar, 3500 Vaerloese (DK); KIBENICH, Annette, 2650 Hvidovre (DK); STROEMAN, Per, 2850 Naerum (DK)
(86) International application number: PCT/EP2016/053664
(87) International publication number: WO 2016/135093

(56) References cited:
- WO-A1-2010/022790
- NEVIANI E ET AL: "ACIDIFICATION ACTIVITY OF THERMOPHILIC LACTOBACILLI UNDER THE TEMPERATURE GRADIENT OF GRANA CHEESE MAKING", JOURNAL OF DAIRY SCIENCE, AMERICAN DAIRY SCIENCE ASSOCIATION, US, vol. 78, no. 6, 1 June 1995 (1995-06-01), pages 1248-1252, XP000518496, ISSN: 0022-0302, DOI: 10.3168/JDS.S0022-0302(95)76744-3

## Description

### FIELD OF INVENTION

The present invention relates to a thermotolerant *Lactobacillus delbrueckii* subsp. *lactis* DSM 32009 strain that can be used in the production of cheese where curd cooking at high temperature (>50°C) is a part of the cheese manufacturing process, such as e.g. Grana and Emmental cheese.

### BACKGROUND OF INVENTION

During cheese manufacture the curd-whey mixture is often heated in order to promote syneresis. The step is referred to as scalding or cooking. The temperature used is different for each cheese variety. For cheeses such as Emmental and Parmesan this temperature is high, usually in the range of 52 to 55°C. Such high temperatures can have a negative consequence on syneresis and cheese quality as they can kill or strongly inhibit the growth of the starter culture, thus resulting in poor acidification of the curd. For these types of cheeses, it is important that the starter culture is composed of thermophilic strains that can survive the high temperature cooking step. Strains that can grow and acidify at high temperature would also be a clear advantage.

NEVIANI E ET AL, JOURNAL OF DAIRY SCIENCE, AMERICAN DAIRY SCIENCE ASSOCIATION, US, vol. 78, no. 6, 1 June 1995 (1995-06-01), pages 1248-1252, XP000518496, ISSN: 0022-0302, DOI: 10.3168/JDS.S0022-0302(95)76744-3 investigates the acidification activity of thermophilic Lactobacilli under the temperature gradient of Grana cheese making.

### SUMMARY OF INVENTION

To develop bacterial strains that are able to acidify milk even at temperatures above 50°C and therefore can be used for cheese making processes with a high temperature cooking step (>50°C) a screening process was designed and executed. The outcome of the screening was surprising as only one strain was able to perform satisfactory in the temperature profile used for the production of whey starter for manufacture of Grana Padano cheese. This strain was identified to be a *Lactobacillus delbrueckii* subsp. *lactis* strain.

It was further observed that only one isolate of the tested strains, *Lactobacillus delbrueckii* subsp. *lactis* can acidify milk at a temperature ≥50°C and consequently perform satisfactory in the temperature profile used for the production of whey starter for manufacture of Grana Padano cheese. The strain is assigned the deposit number: DSM 32009. In accordance with the surprising finding, an aspect of the present invention relates to the use of a *Lactobacillus delbrueckii* subsp. *lactis* DSM 32009 strain, that can acidify milk at high temperature, in a cheese making process that involves a high temperature cooking step (>50°C).

### DETAILED DESCRIPTION OF THE INVENTION

The present invention relates to a process for producing cheese, which comprises adding to milk a starter culture comprising an acidifying strain belonging to the genus *Lactobacillus* and heating the mixture to a temperature (or maintaining the temperature) in the range of 45°C to 65°C, such as in the range 50°C to 60°C or in the range 53-54°C, such as e.g. 54°C.

In a related aspect, the present invention relates to a process for producing cheese, which comprises adding to milk a starter culture comprising an acidifying strain belonging to the genus *Lactobacillus* and heating the mixture to a temperature (or maintaining the temperature) in the range of 45°C to 65°C, such as in the range 50°C to 60°C or in the range 53-54°C, such as e.g. 54°C and adding to mixture one or more bacterial strains or cultures, such as e.g. adjunct cultures and optionally one or more coagulants.

The acidifying *Lactobacillus* is able to acidify the milk by at least 0.4 pH units in 2.5 hours at a temperature around 50 °C when inoculated with a 3% whey starter culture.

According to the invention, the acidifying strain of *Lactobacillus* is a *Lactobacillus delbrueckii* subsp. *lactis* strain deposited as DSM 32009, or a mutant thereof.

In a related aspect, the starter culture further comprise one or more bacterial strains selected from the list consisting of: *Lactococcus lactis, Leuconostoc mesenteroides, Pediococcus pentosaceus, Lactobacillus casei, Lactobacillus paracasei, Streptococcus ther mophilus, Enterococcus faecium, Lactobacillus delbrueckii* subsp. *bulgaricus, Lactobacillus delbrueckii* subsp. *lactis, Lactobacillus helveticus* spp, *Lactobacillus acidophilus* or *Propionibacterium.*
In yet a related aspect, the one or more coagulants is selected from the list comprising: bovine derived coagulants or variants thereof, microbial derived coagulants or variants thereof, camel derived coagulants or variants thereof, rennets and chymosins.
In yet a related aspect, the starter culture is a whey starter culture and in related embodiments the whey starter culture may be subject to the physical conditions comprising: 1) a temperature drop from around 48°C to around 39°C in 9 hours, 2) a constant temperature of around 39°C for around 8 hours, 3) cooling and storing at around 16°C until it is used for milk acidification.

In yet a related aspect, the starter culture and or one or more bacterial strains is added as a direct vat set (DVS) culture, such as e.g. a frozen direct vat set (F-DVS) culture or a freeze-dried direct vat set (FD-DVS) culture. The DVS culture may be added to a whey starter and/or directly to the milk. If added to the whey, the acidifying strain belonging to the genus *Lactobacillus* may be added in an amount of at least 5x10¹⁰ CFU/500liter whey.

The process of present invention may further comprise adding one or more bacterial strains or cultures, such as e.g. adjunct cultures such as e.g. *Propionibacterium* to the milk.

The process of present invention may further comprise the necessary actions needed to produce a cheese. Hence the present invention also relate to a cheese (including a low-fat cheese) obtainable by the process of any of the aspect disclosed herein. More specifically the may be a Grana type cheese such as e.g. a Grana Padano, Parmigiano Reggiano or alternatively of the Emmental type.

As disclosed herein, the invention also relate to an acidifying *Lactobacillus delbrueckii* subsp. *lactis* strain deposited as DSM 32009 or any mutant thereof. If commercially relevant, the acidifying strain of *Lactobacillus* may be modified by the act of man.
The *Lactobacillus* of present invention may be forming part of a mixture with desired properties. Hence the present invention also relates to a composition comprising the acidifying *Lactobacillus delbrueckii* as described in present disclosure.
Such composition may comprise one or more excipients such as e.g. polyhydroxy compounds such as e.g. sugars, polyalcohols and glycols and their derivatives, trehalose and dimethyl sulfoxide (DMSO).

The composition may be a direct vat set (DVS) starter culture composition, such as e.g. a frozen direct vat set (F-DVS) starter culture composition or a freeze-dried direct vat set (FD-DVS) starter culture composition. The DVS starter culture may be added to a whey starter. In a related aspect, the DVS starter culture composition comprises at least 1x10¹⁰ CFU/g.

In a related aspect, the starter culture is added to the milk in an amount sufficient to facilitate acidification of the milk by at least 0.4 pH units in 2.5 hours at a temperature around 50°C.

As illustrated by the vast aspects described herein, the invention also relate to the use of the acidifying *Lactobacillus delbrueckii* subsp. *lactis* strain DSM32009 in a process for making cheese, such as e.g. Grana or Emmental type cheese as well as a composition as described in the aspects herein, in a process for making cheese, such as e.g. Grana or Emmental type cheese.

### DEFINITIONS

By the term "milk" is understood a composition comprising lacteal secretion obtained from any mammal, such as an animal of a species belonging to the subfamily *Bovinae* (which includes the domestic cow (*Bos taurus*) and buffalo); an animal of a species belonging to the subfamily *Caprinae* (which includes goat and sheep); or an animal of the species *Camelidae* (which includes camels). Optionally the milk is acidified, e.g. by addition of an acid (such as citric, acetic or lactic acid) or by addition of an acid producing microorganism. The milk may be raw or processed, e.g. by filtering, sterilizing, pasteurizing, homogenizing, fractionating (e.g. reducing the fat content of the milk), etc., or it may be reconstituted dried milk. An important example of "milk" according to the present invention is pasteurized cow's milk. It is understood that the milk may be acidified, mixed or processed before, during and/or after the adding of bacterial cultures. The term "milk" also comprises milk with protein, calcium or other additives added.

The term "cheese" refers to a product prepared by contacting optionally acidified milk (e.g. by means of a lactic acid bacterial culture) with a coagulant, and draining the resultant curd. Cheeses and their preparation are described in e.g. Cheese and Fermented Milk Foods, by Frank V. Kosikowski.

The term "high temperature cooking step" should be understood as a cooking step with a temperature >50°C. This is typical for e.g. cheese of the Grana and Emmental type.

As used herein the term 'modified by the act of man' should be understood as a modification of the *Lactobacillus* strain aiming to increase the commercial applicability of said *Lactobacillus* strain. Such methods comprise increasing the thermo-tolerance by growing the strain at elevated temperatures such as e.g. >50°C, subjecting the strain to heat-stress to cure antibiotic resistance, such as e.g. kanamycin resistance if relevant. Other modifications made by man could be optimization for accelerated germination or acidification.

As used herein the term "lactic acid bacterium" designates a gram-positive, microaerophilic or anaerobic bacterium, which ferments sugars with the production of acids including lactic acid as the predominantly produced acid. The industrially most useful lactic acid bacteria are found among *Lactococcus* spp., *Streptococcus* spp., *Lactobacillus* spp., *Leuconostoc* spp., *Pediococcus* spp.. Additionally, lactic acid producing bacteria belonging to the group of the strict anaerobic bacteria, *bifidobacteria,* i.e. *Bifidobacterium* spp., which are frequently used as food cultures alone or in combination with other lactic acid bacteria, are generally included in the group of lactic acid bacteria.

The term "starter culture" relates to any bacterial culture that is suitable for use in milk acidification, especially lactic acid bacteria such as *Bifidobacteria, Lactobacilli, Lactococci, Leuconostocs, Micrococci, Streptococci and Pediococci.* It will be appreciated that the term starter culture may encompass a culture containing a single strain of bacterium, or more than one bacterial strain. The term may also include genetically modified organisms (GMO's). In any event, the term is well known in the art and the invention extends equally to all known starter cultures. The term includes bacterial cultures containing a strain of a genus selected from the group consisting of *Lactococcus, Lactobacillus, Micrococcus, Leuconostoc, Pediococcus, Streptococcus, Enterococcus,* etc. such as a strain of a species selected from the group consisting of: *Lactococcus lactis (incl. Lactococcus subsp. lactis* subsp. *lactis, Lactococcus* lactis subsp. *cremoris,* and *Lactococcus lactis subsp. lactis* biovar. *diacetylactis*), *Leuconostoc mesenteroides* (incl subsp. *cremoris*), *Pediococcus pentosaceus, Lactobacillus casei* (incl. subsp. *casei*) and *Lactobacillus paracasei* (incl. subsp. *paracasei*), *Streptococcus thermophilus, Enterococcus faecium, Lactobacillus helveticus, Lactobacillus delbrueckii* subsp. *bulgaricus, Lactobacillus delbrueckii* subsp. *subsp. lactis* and *Lactobacillus acidophilus.* Other useful bacterial species are *Bifidobacterium* species including *B. bifidum, B. lactis* and *B. longum, Streptococcus faecium, Leuconostoc lactis.*

As previous mentioned, lactic acid bacteria are essential in the making of nearly all fermented milk products e.g. cheese, and they are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product. Such cultures are in general referred to as "starter cultures" or "starters".

Commonly used starter culture strains of lactic acid bacteria are generally divided into mesophilic organisms having optimum growth temperatures at about 30 to 35°C and thermophilic organisms having optimum growth temperatures in the range of about 40 to about 45°C. Typical organisms belonging to the mesophilic group include *Lactococcus lactis* subsp. *lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis, Lactobacillus casei* subsp. *casei* and *Lactobacillus paracasei* subsp. *paracasei.* Thermophilic or thermotolerant lactic acid bacterial species include as examples *Streptococcus thermophilus, Enterococcus faecium, Lactobacillus delbrueckii* subsp. *lactis, Lactobacillus helveticus, Lactobacillus delbrueckii* subsp. *bulgaricus, Lactobacillus delbrueckii subsp. lactis* and *Lactobacillus acidophilus.*
Additionally, species of *Propionibacterium* are used as dairy starter cultures, in particular in the manufacture of cheese. As reflected by the teachings disclosed in e.g. Rossetti et al. 2008, several studies have been made on the microbial composition of starter cultures in cheese making. Hence the person skilled in the art of making cheese (and in particular Grana) will know the composition of the starter-culture to which the strain according to present invention may be added.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### DRAWINGS

Figure 1 depicts the temperature profile used for the generation of the whey starter culture containing the *Lactobacillus delbrueckii subsp. lactis* DSM 32009, cf. the example 1.
Figure 2 depicts the evolution of the pH in the whey starter solution during the incubation step as described in Figure 1.
Figure 3 depicts the temperature profile used for the milk acidification using a whey starter containing the *L. delbrueckii subsp. lactis.*
Figure 4 depicts the evolution of the pH in milk inoculated with a whey starter solution with and without the *L. delbrueckii subsp. lactis.*

### EXPERIMENTAL

### Example 1: Acidification of milk by a whey starter containing the Lactobacillus delbrueckii subsp. lactis DSM 32009 strain when a temperature gradient with a high temperature cooking step is applied.

### a) Generation of whey starter containing the Lactobacillus delbrueckii subsp. lactis DSM 32009 strain.

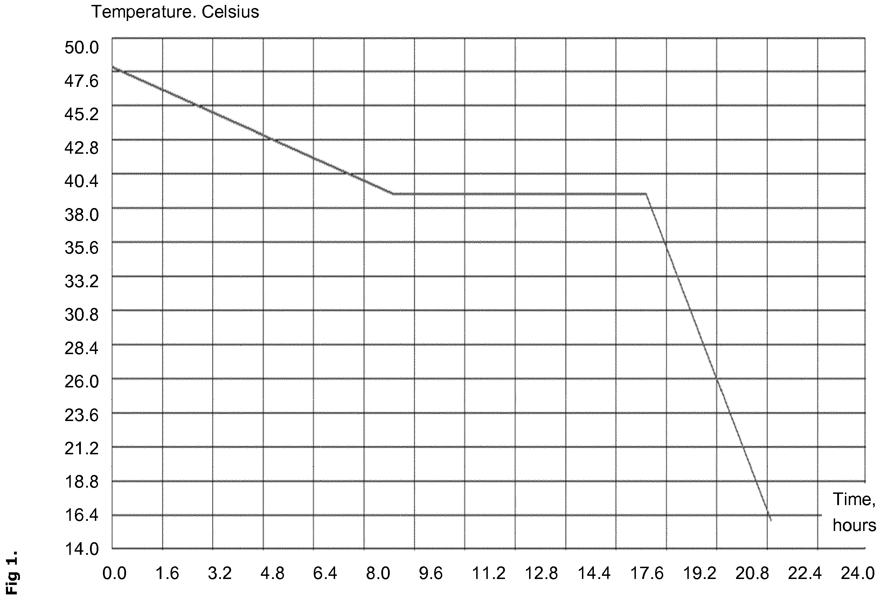

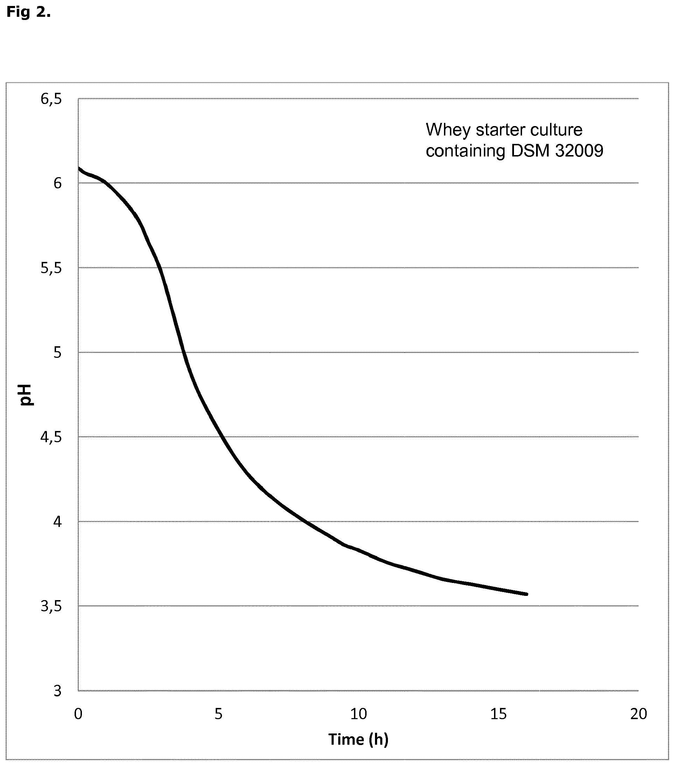

Whey starter media consisted of a 7% sweet whey powder solution in water. After dissolution of the whey powder, the whey media was pasteurized for 20 minutes at 90°C. 200ml of sweet whey media were inoculated with the whey starter culture comprising a mix of *Lactobacillus* spp and *Streptococcus thermophilus* with and without DSM 32009 The starter was incubated as described in Figure 1. pH was measured continuously with pH probes connected to a datalogger as shown in Figure 2. Temperature compensation was used to secure accurate pH measurement throughout the temperature gradient. The whey starter culture containing the *Lactobacillus delbrueckii subsp. lactis* DSM 32009 strain acidified to pH 3.5 in 16 hours.

Figure 1 shows the temperature profile used for the generation of the whey starter culture. This temperature profile simulates the temperature profile used for the production of whey starter for manufacture of Grana Padano cheese. The temperature profile consists of:
- A temperature drop from 48°C to 39°C in 9 hours
- A constant temperature of 39°C for 8 hours
- The whey starter is then cooled and maintained at 16°C until it is used for milk acidification.

Figure 2 shows the evolution of the pH in the whey starter solution during the incubation step as described in Figure 1.

### b) Acidification of milk with a whey starter containing the Lactobacillus delbrueckii subsp. lactis DSM 32009 strain

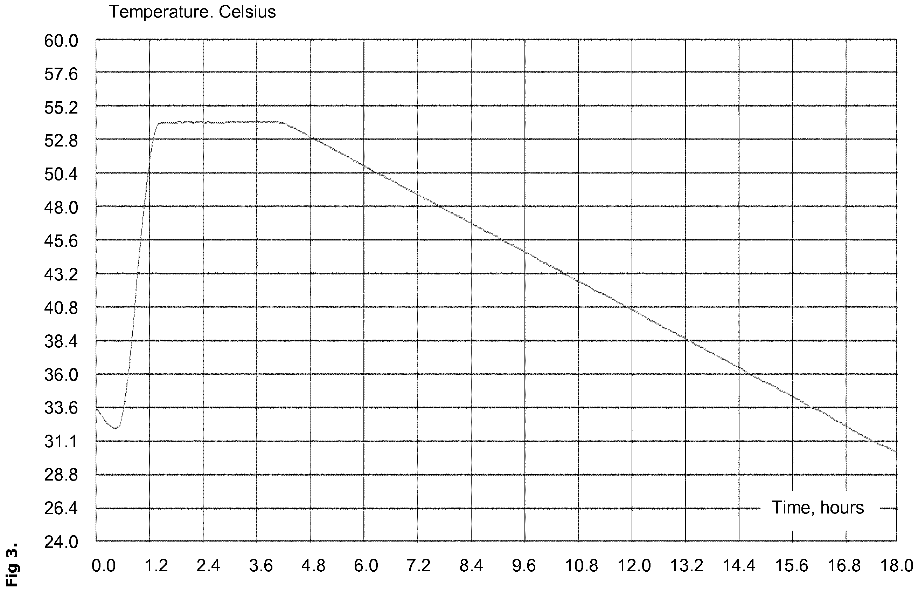

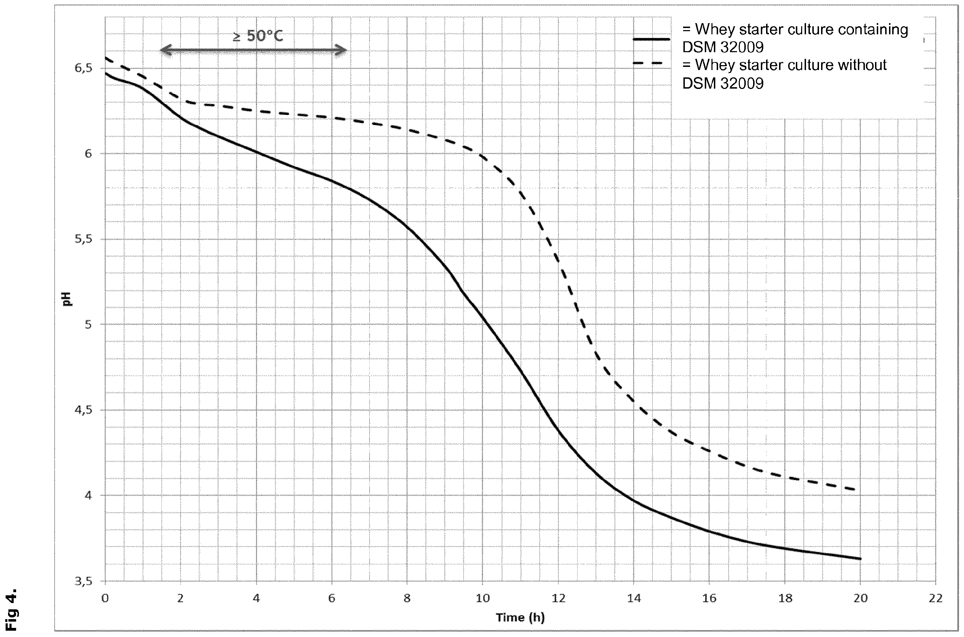

Milk used for acidification experiment was prepared from 9.5 % skim milk powder rehydrated in water and subsequently heat treated at 140 °C/8 sec and 100 °C/30 min. Milk was inoculated with 3% of the whey starter prepared as described in Example 1.a. The pH was measured continuously with pH probes connected to a datalogger as shown in Figure 4. Temperature compensation was used to secure accurate pH measurement throughout the temperature gradient. Incubation temperature was as described in Figure 3. During the time where the temperature is above 50°C, the whey starter culture containing the *Lactobacillus delbrueckii subsp. lactis* DSM 32009 strain decreased the pH of the milk from pH 6.30 to pH 5.80 (deltapH=0.50 pH units). The whey starter culture without the *Lactobacillus delbrueckii subsp. lactis* DSM 32009 strain decreased the pH of the milk from pH 6.40 to pH 6.20 (deltapH=0.2 pH units). Moreover, it is observed that the whey starter culture containing the *Lactobacillus delbrueckii subsp. lactis* DSM 32009 strain continues to lower the milk pH after the cooking step while the whey starter culture without the *Lactobacillus delbrueckii subsp. lactis* DSM 32009 strain has a longer lag phase with no or very little pH drop. As a comparison, the milk acidified with the whey starter culture containing the *Lactobacillus delbrueckii subsp. lactis* DSM 32009 strain reaches pH 5.0 in 10 hours while the milk acidified with the whey starter culture without the *Lactobacillus delbrueckii subsp. lactis* DSM 32009 strain reaches pH 5.0 in 12.5 hours.

Figure 3 shows the temperature profile used for the milk acidification using a whey starter containing the *Lactobacillus delbrueckii subsp. lactis* DSM 32009 strain. This temperature profile simulates the temperature profile used for the manufacture of Grana Padano cheese. The temperature profile consists of:
- A constant temperature of 32°C for 30 min
- A temperature increase to 54°C and kept constant for 3 hours
- A linear temperature decrease to 30°C

Figure 4 shows the evolution of the pH in milk inoculated with a whey starter solution containing or not the *Lactobacillus delbrueckii subsp. lactis* DSM 32009 strain. Temperature gradient is as described in Figure 3.

### REFERENCES

Rosetti, L., Fornasari, M. E., Gatti, M., Lazzi, C., Neviani, E. and Giraffa, G. Grana Padano cheese whey starters: Microbial composition and strain distribution. International Journal of Food Microbiology 127 (2008) pp. 168-171.

## Claims

1. An acidifying Lactobacillus delbrueckii strain, wherein the Lactobacillus delbrueckii strain is an acidifying Lactobacillus delbrueckii subsp. lactis strain desposited as DSM 32009 or a mutant of the acidifying Lactobacillus delbrueckii subsp. lactis strain deposited as DSM 32009.

2. An acidifying Lactobacillus delbrueckii strain according to claim 1, wherein the Lactobacillus delbrueckii strain is an acidifying Lactobacillus delbrueckii subsp. lactis strain desposited as DSM 32009.

3. A process for producing cheese, which comprises adding to milk:
- a starter culture comprising an acidifying strain belonging to the genus *Lactobacillus* of any of claim 1-2 and
heating the mixture to a temperature in the range of 45 to 65°C.

## Patentansprüche

1. Acidifizierender Lactobacillus delbrueckii Stamm, wobei der Lactobacillus delbrueckii Stamm ein acidifizierender Lactobacillus delbrueckii subsp. Lactis Stamm, der als DSM 32009 hinterlegt ist, oder ein Mutant des acidifizierenden Lactobacillus delbrueckii subsp. Lactis Stamms, der als DSM 32009 hinterlegt ist, ist.

2. Acidifizierender Lactobacillus delbrueckii Stamm nach Anspruch 1, wobei der Lactobacillus delbrueckii Stamm ein acidifizierender Lactobacillus delbrueckii subsp. Lactis Stamm ist, der als DSM 32009 hinterlegt ist.

3. Verfahren zum Herstellen von Käse, welches das Hinzufügen des Folgenden zu Milch umfasst:
- eine Starterkultur eines acidifizierenden Stamms, der zu der Gattung Lactobacillus nach einem der Ansprüche 1-2 gehört und
Erhitzen der Mischung auf eine Temperatur in dem Bereich von 45 bis 65 °C.

## Revendications

1. Souche acidifiante Lactobacillus delbrueckii, dans laquelle la souche Lactobacillus delbrueckii est une souche acidifiante Lactobacillus delbrueckii de la sous-espèce lactis déposée en tant que DSM 32009 ou un mutant de la souche acidifiante Lactobacillus delbrueckii de la sous-espèce lactis déposé en tant que DSM 32009.

2. Souche acidifiante Lactobacillus delbrueckii selon la revendication 1, dans laquelle la souche Lactobacillus delbrueckii est une souche acidifiante Lactobacillus delbrueckii de la sous-espèce lactis déposée en tant que DSM 32009.

3. Procédé de production de fromage, qui comprend l'ajout au lait :
- d'une culture de départ comprenant une souche acidifiante appartenant au genre *Lactobacillus* selon l'une quelconque des revendications 1 à 2 et
le chauffage du mélange à une température comprise entre 45 et 65 °C.
